# EUROPEAN PATENT SPECIFICATION

(11) **EP 0 577 684 B1**
(45) Date of publication and mention of the grant of the patent: **14.05.1997**
(21) Application number: 92907545.5
(22) Date of filing: 30.03.1992
(51) Int. Cl.: G01N 21/31, A61B 5/00, G01N 33/48

(54) **METHOD AND APPARATUS FOR GLUCOSE CONCENTRATION MONITORING**
VERFAHREN UND VORRICHTUNG ZUR ÜBERWACHUNG DER GLUKOSEKONZENTRATION
PROCEDE ET APPAREIL DE CONTROLE DE LA CONCENTRATION DE GLUCOSE

(30) Priority: 28.03.1991 GB 9106672
(43) Date of publication of application: 12.01.1994
(73) Proprietor: JOHNSON & JOHNSON PROFESSIONAL PRODUCTS LIMITED, Bracknell, Berkshire RG12 2AT (GB)
(72) Inventor: EAGLES, Obreon, Swansea, Wales SA6 6DD (GB); WALL, Peter, Ogmore Valley, Mid Glamorgan CF32 7ET (GB); PARKER, Dawood, Whitland, Dyfed SA34 0LG (GB)
(74) Representative: Mercer, Christopher Paul
(86) International application number: GB9200572
(87) International publication number: WO9217765

(56) References cited:
- EP-A- 0 160 768
- EP-A- 0 401 453
- WO-A-90/07905
- WO-A-91/15990
- US-A- 4 882 492
- PATENT ABSTRACTS OF JAPAN vol. 8, no. 42 (P-256)(1479) 23 February 1984 & JP-A-58 193 438 ( TOUA DENPA KOGYO K K ) 11 November 1983

## Description

This invention relates to a method and apparatus for measuring the concentration of glucose in an aqueous solution.

The brain uses glucose almost exclusively as an energy source, and it is therefore extremely important to maintain blood glucose levels within a very narrow range. Under normal conditions the levels of glucose in the blood stream are maintained in an elaborate series of mechanisms and feedback loops. These serve to counter fluctuations in glucose and restore it to the correct level.

In normal patients high levels of glucose trigger insulin release into the blood stream from the pancreas and conversely low levels suppress its release, thus regulating the normal blood glucose level.

Diabetes mellitus is caused by a lack of insulin in the patient which leads to a chronically raised blood glucose level causing unpleasant short term symptoms such as frequent urination and thirst, and more serious long term problems in the form of damage to the kidneys, blood vessels, nerves and eyes.

Low levels of blood glucose cause a condition known as hypoglycaemia which results in short term mental confusion, and, if prolonged, coma and even death.

A known method of detecting and treating diabetic patients involves the analysis of blood withdrawn from the patient. This involves the patient pricking a finger with a sterile lance to produce a sample of capillary blood which is placed on a test strip impregnated with appropriate chemicals to produce a colour change, the intensity of which is compared with a printed standard card. A more recent development has been a technique (EXACTECH Pen) which gives a digital readout of blood glucose concentration a few seconds after a sample has been placed on the disposable thin film printed sensor.

If diabetes is diagnosed, insulin may be prescribed. Since the glucose level in each individual is variable, in the absence of continuous measurements of blood glucose, it is impossible to give insulin which is appropriate at all times to the physiological need. As a consequence, diabetic patients still have periods of high blood glucose levels. On the other hand, administration of excess insulin can result in low blood glucose.

Near infrared spectrometric techniques are known for measuring the concentration of particular substances in blood. For example, European patent application 0160768 describes a reflectance technique using pre-selected test and reference wavelengths. U.S. patent 4655225 describes a technique utilising spectrophotometric analysis of near infra-red test wavelengths to ascertain the presence of glucose. Near infra-red absorption of glucose is described with peak wavelength for glucose adsorption of 2098nm and a reference wavelength of 1100nm. Also PCT patent application WO90/07905 describes a technique where 980nm is used as the peak wavelength for glucose detection.

The selection of the wavelength of the radiation is critical for the following reasons:
1. The concentration of glucose averages only about 0.1% by weight of blood serum. An accuracy of 50ppm is required for any meaningful measurement of glucose concentration.
2. Other components in the blood absorb in the near infra-red region and could interfere with the glucose measurement, e.g. the concentrations of serum proteins are significantly higher than glucose, therefore, compounding the interference problem.
3. Absorption measurements of aqueous solutions are difficult because of the strong background absorption of water. This strong absorption severely limits the application and use of a conventional spectrometer in solving this problem.

The fact that water is a major constituent of tissue is a significant factor in choosing the wavelength at which to measure the concentration of glucose in blood. Water exhibits strong absorption bands in the near infra-red with maxima around 1130nm, 1420nm and 1910nm. Transmission windows for water open at around 1250nm, 1600nm and 2150nm. For these reasons the wavelength chosen should be as low as possible and should be within the transmission windows for water.

We have now devised an improved technique and apparatus for measuring the concentration of blood glucose. According to a first aspect of the invention, there is provided apparatus as defined in the claims for determining the concentration of glucose in a test medium of blood, which apparatus comprises:
(a) a first radiation source for generating a first beam of electromagnetic radiation of a wavelength substantially in the bandwidth 1500 to 1700nm;
(b) a second radiation source for generating a second beam of electromagnetic radiation of a second wavelength in the bandwidth 1200 to 1400nm;
(c) detector means arranged to detect electromagnetic radiation from said first and second radiation beams;
(d) means arranged to direct said first and second electromagnetic radiation beams along respective paths through a test medium of said blood to said detector means; and a heater means capable of elevating the temperature of the test medium.

The detector means is spaced from the first and second radiation means such that the blood to be tested (i.e. the test medium) which may be contained in a transparent-walled container, or alternatively, in part of the body (such as a finger), which may be interposed between the detector means and first/second radiation sources.

Advantageously, the first wavelength is in the bandwidth 1547nm to 1577nm, and is more preferably of 1547 to 1557nm.

It is preferred that the second wavelength is in the bandwidth 1295nm to 1305nm, and is more preferably of wavelength substantially 1300nm.

It is preferred that pulsation means is provided for the apparatus, such that at least one of the radiation beams may be pulsed. Preferably, the pulsation means enables both the first and second radiation beams to be pulsed, advantageously alternately.

Pulsation of the beams enables high power radiation to be used, and thereby increases the ability of the radiation beams to penetrate sufficiently through the test medium to the detector means. This is particularly the case where the apparatus is used for in vivo measurements utilising for example a person's finger containing the test medium (i.e. blood).

It is preferred that the respective paths of the radiation beams are substantially rectilinear through the test medium. Advantageously the paths of the radiation beams are effectively co-linear through the test medium.

The first and second radiation sources may be laser devices (preferably monochromatic). More preferably, laser diodes are arranged to emit respective laser beams of the required frequency.

Alternatively, the radiation sources may be combined in a single laser (or laser diode) source arranged to emit two radiation beams (corresponding to the first and second radiation beams) of the required frequency.

The detector means may comprise a discrete detector for each radiation beams; however it is preferred that the detector means is in the form of a unitary detector arranged to detect radiation from both the first and second beams of electromagnetic radiation.

The detector means preferably produces electrical output signals dependent on the intensity of the electromagnetic radiation impinging on the detector representing the intensity of the first and second radiation means. Advantageously, signal processing and conditioning means is provided arranged to process the output signals from the detector means and provide a value corresponding to the difference in the intensities of the respective radiation beams impinging on the detector means, thereby enabling a value of glucose absorption (and hence glucose concentration) to be found. Preferably, the detector is in the form of a photodiode, for example, a germanium photodiode, and is preferably coupled via appropriate electrical circuitry to the signal processing and conditioning apparatus such that a value of glucose concentration in the blood may be obtained.

The apparatus further comprises heater means arranged to elevate the temperature of the blood under test. Advantageously, the heater means is capable of elevating the blood temperature to 40 degrees Celsius.

Typically collimator means is provided for the apparatus, arranged to direct the first and second electromagnetic radiation beams along their respective paths through the test medium.

The collimator means may comprise a lens and/or prism arrangement arranged to direct the radiation beams along effectively co-linear paths.

In a preferred embodiment, optical waveguides may comprise at least a part of the collimator means. In particular, in this embodiment optical fibre waveguides may be used in close side by side arrangement such that the first and second radiation beams effectively follow the same (i.e. co-linear) path through the test medium.

In an embodiment of the apparatus according to the first aspect of the invention, the apparatus is adapted for in vitro determination of glucose concentration of a sample of blood. In this embodiment, the apparatus preferably further comprises a walled receptacle within which the sample may be contained. The receptacle walls preferably comprise a transparent substance, and advantageously a spectrophotometric glass cuvette may be used. Advantageously, a cover is also provided for the receptacle, arrangeable to exclude ambient light from entering the receptacle.

In a second embodiment according to the first aspect of the invention, the apparatus is adapted for use in non-invasive measurement of glucose concentration. In this embodiment, it is preferred that a sleeve member suitable for receiving a finger of a patient is provided as a housing for at least some of the components of the apparatus, and particularly the detector means. Advantageously, the sleeve is also arranged to house the collimator means, and also the first and second radiation sources where practicable, e.g. where laser diodes are used.

According to a second aspect of the invention, there is provided a method as defined in the claims of determining the concentration of glucose in a test medium of blood, which method comprises directing a first radiation beam of a wavelength substantially in the bandwidth 1500 to 1700nm along a first path from a source to radiation detection means; directing a second radiation beam of a wavelength substantially in the band width 1200 to 1400nm, along a second path from a source to said radiation detection means, said first and second paths passing through said test medium in a region intermediate said source(s) and said radiation detection means.

It is preferred that the respective paths of the radiation beams are rectilinear as they pass through the test medium, advantageously the respective paths of the radiation beams are effectively co-linear as they pass through the test medium.

It is preferred that at least one of the first and second radiation beams is pulsed such that an intermittent beam of radiation is provided. Preferably, both the first and second radiation beams are pulsed alternately.

The temperature of the blood is elevated, preferably to about 40 degrees Celsius, before, or during, determination of the glucose concentration by the method according to the invention.

The invention will now be further described in a particular embodiment, by way of example only, with reference to the accompanying drawings, in which:

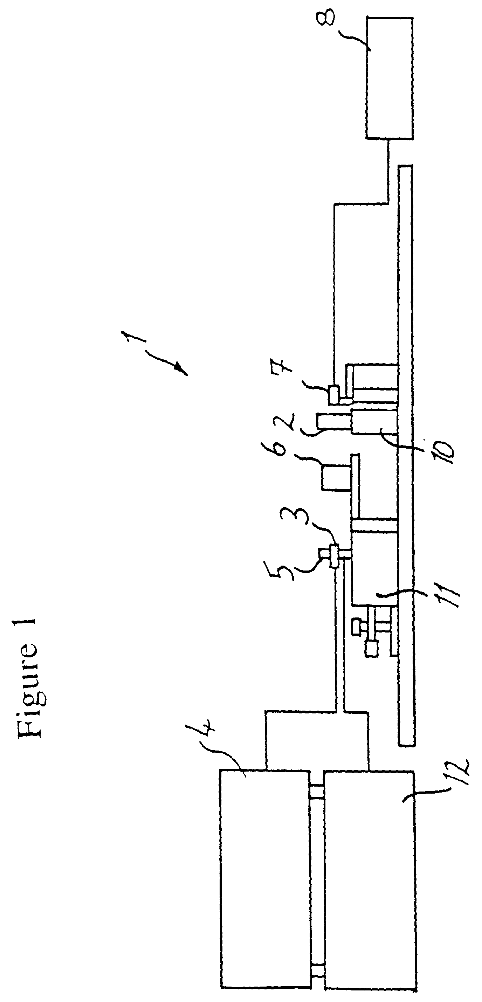

Figure 1 is a schematic representation of apparatus according to the invention which is suitable for carrying out the method according to the invention;
Figure 2 is a schematic plan view of part of the apparatus of Figure 1; and
Figure 3 is a schematic representation of alternative apparatus according to the invention.

Referring to the drawings, the apparatus generally designated 1 is arranged to determine the concentration of glucose in a sample of blood contained in a high precision spectrophotometric glass cuvette 2.

Two laser diodes 3, are driven by standard laser driver circuitry 4. Of the laser diodes 3 used in the apparatus, one is selected to emit a beam of wavelength of 1552nm (which is absorbed by glucose and therefore acts as the test beam), and one selected to emit a beam of wavelength of 1310nm (which is not absorbed by glucose and therefore acts as a reference beam).

The operating temperature of the laser diodes can be controlled by the use of a Peltier-driver 12. The operating temperature of the diodes can be varied to change to emitted wavelengths of the respective beams by ± 5nm. Accordingly the apparatus operates optimally at constant temperature.

The laser diodes 3 are mounted one either side of polished reflecting prism 5. Light emitted from each diode 3 is reflected by the prism 5 through a collimating lens 6. Thus prism 5 and collimating lens 6 comprise an optical system which directs and focuses the beams emitted from the laser diodes 3 such that they follow the same rectilinear path (i.e. the two paths are co-linear) throughout the blood sample in the glass cuvette 2.

The alignment of the respective laser diodes 3 with the optical system is controlled by XYZ axis Micropositioner 11.

Alternatively, the beams may be directed from respective laser diodes by means of fibre optic waveguides arranged in close side by side arrangement at their emitting ends. Since the diameter of the optical fibres would be of the order of 400 micrometres, the laser beams would follow effectively the same co-linear path.

Furthermore, the separate laser diodes 3 may be replaced by a single semi-conductor device such as a single diode arranged to emit separate beams of the required wavelength.

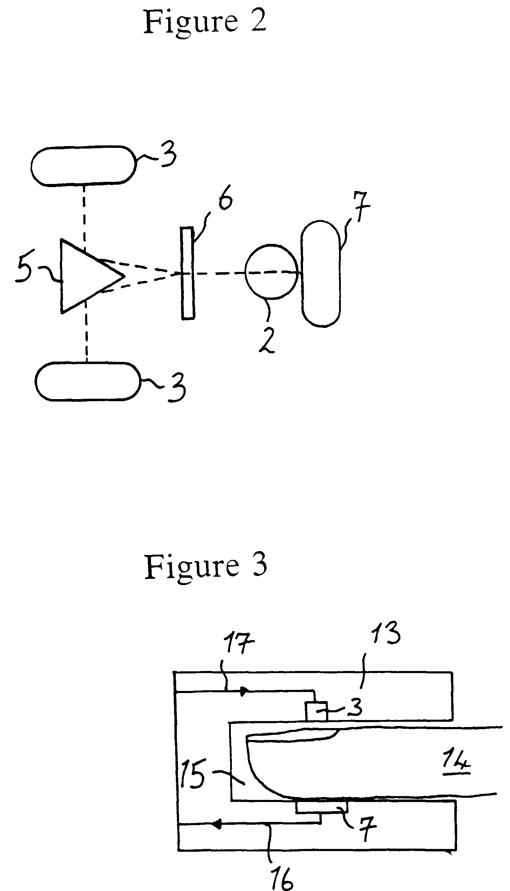

To ensure that the two beams emitted from respective laser diodes 3 are not superposed whilst following the same path best shown in Figure 2 through the blood sample, the laser diode drive circuitry 4 is arranged to supply power to each diode 3 cyclicly such that the respective laser beams emitted thereby are pulsed alternately. The pulsing of the beams enables higher intensity beams to be used than would ordinarily be possible with a "constant" beam, without causing damage to the structure of the blood. The ability to use high intensity beams ensures that the light beam travels right through the test sample in the glass cuvette 2.

The laser diodes used in this instrumentation may be pulsed with a duty cycle of 0.1 milliseconds, and a pulse duration of 4 microseconds. These parameters are dependent on laser diodes characteristics and may be modified accordingly.

A photodiode detector 7 is arranged adjacent the glass cuvette 2 to collect light from the laser beams which is transmitted through the blood sample in the glass cuvette 2. The photodiode detector may be a germanium diode; alternatively an Indium Gallium Arsenide detector may be used. The photodiode detector 7 produces an electrical output signal proportional to the intensity of light detected, and this signal is relayed via appropriate conventional electronic circuitry to signal processing and conditioning apparatus 8 arranged to give a digital output value related to the intensity of light detected by the photodiode detector 7.

In use, since the diodes 3 are pulsed alternately, the respective laser beams generated pass alternately along the same path through the blood sample contained in the glass cuvette 2. The light of the respective beams is therefore alternately transmitted to the photodiode detector 7 where alternate respective electrical output signals are generated.

The light beam of wavelength 1552nm is highly absorbed by glucose in solution in the blood sample, and therefore the transmitted light of this wavelength is related to the concentration of glucose within the blood sample. The light beam of wavelength 1310nm is not absorbed by glucose in solution in the blood sample, and therefore the intensity of this wavelength detected by the photodetector 7 relates to the "background" absorption of water and no other constituents the blood. Appropriate comparison and processing of these alternating respective output signals by the signal processing and conditioning apparatus 8 enables a value for glucose concentration in the blood sample to be obtained.

Unexpectedly, it was found that if the temperature of the blood in the cuvette was elevated to around 40°C, the amplitude of the light beams transmitted to the photodetector 7 increased considerably for the test beam. This is extremely beneficial in terms of sensitivity of measurement of glucose concentration in the blood sample, and a cuvette heater 10 was therefore incorporated in the apparatus. However, to meaningfully compare sample with sample, the temperature at which the measurement is made has to be constant and identical in each case. Without this heating effect the resolution is not good enough to differentiate accurately and reproducibly between different glucose-containing samples within the ranges of clinical significance.

Referring to Figure 3, an alternative embodiment of apparatus is shown adapted for in vivo use. An electrically heated sleeve (or housing) 13 defines a finger receiving cavity 15 within which the patients finger 14 is received and elevated to a temperature of about 40°C. A single semi-conductor laser diode 3 is located in the sleeve 13 and emits the two radiation beams of the "test" and "reference" beam wavelengths (defined above) respectively. The beams penetrate and pass through the user's finger where they are received at the detector 7 which produces respective output signals which are then sent to appropriate signal processing apparatus (not shown) along appropriate circuitry 16. The laser diode 3 is connected to driver and pulsation apparatus (not shown) by appropriate circuitry 17.

## Claims

1. Apparatus (1) adapted for the non-invasive measurement of glucose concentration in a patient's blood comprising:
a first radiation source (3) for generating a first beam of electromagnetic radiation of a wavelength substantially in the bandwidth 1500 to 1700nm;
a second radiation source (3) for generating a second beam of electromagnetic radiation of a second wavelength in the bandwidth 1200 to 1400nm;
detector means (7) arranged to detect electromagnetic radiation from said first and second radiation beams;
means (5,6) arranged to direct said first and second electromagnetic radiation beams along respective paths through a test medium of said blood to said detector means (7);
characterised in that a heater means (10) is provided capable of elevating the temperature of the test medium.

2. Apparatus (1) according to claim 1, wherein the respective paths of the radiation beams are substantially rectilinear through said test medium.

3. Apparatus (1) according to claim 2, wherein the paths of the radiation beams are effectively co-linear through said test medium.

4. Apparatus (1) according to any preceding claim, wherein pulsation means is provided for the apparatus (1), such that at least one of the radiation beams is pulsed.

5. Apparatus (1) according to claim 4, wherein both of the radiation sources (3) are pulsed.

6. Apparatus (1) according to claims 4 and 5, wherein the pulsation means is arranged to pulse the first and second radiation beams alternately.

7. Apparatus (1) according to any preceding claim, wherein the first radiation source (3) is arranged to generate a wavelength in the bandwidth 1547nm to 1577nm.

8. Apparatus (1) according to any preceding claim, wherein the second radiation source (3) is arranged to generate a wavelength in the bandwidth 1295nm to 1305nm.

9. Apparatus (1) according to any preceding claims, wherein the first and second radiation sources (3) are laser devices.

10. Apparatus (1) according to claim 9, wherein the first and second laser devices are laser diodes.

11. Apparatus (1) according to claims 9 or 10, wherein a single said laser device is provided to emit both the first and second radiation beams.

12. Apparatus (1) according to any preceding claim, wherein the detector means (7) is in the form of a unitary detector arranged to detect radiation from both the first and second beams of electromagnetic radiation.

13. Apparatus (1) according to any preceding claim, wherein the detector means (7) is arranged to produce an electrical output signal dependent on the intensity of the electromagnetic radiation impinging on the detector (7).

14. Apparatus (1) according to claim (13), wherein the detector (7) is a photodiode.

15. Apparatus (1) according to any preceding claim, wherein collimator means is provided arranged to direct the first and second radiation beams along their respective paths through the test medium.

16. Apparatus (1) according to claim 15, wherein the collimator means comprises an optical lens (6) and/or prism (5) arranged to direct the first and second electromagnetic radiation beams along their respective paths.

17. Apparatus (1) according to claim 15, wherein the collimator means comprises optical waveguide means.

18. Apparatus (1) according to any preceding claim, which further comprises a walled receptacle (2) within which the test medium is contained.

19. Apparatus (1) according to any one of claims 1 to 17, which further comprises a sleeve member (13) shaped and dimensioned to receive a finger (14) of a patient.

20. Apparatus (1) according to claim 19, wherein the sleeve member (13) is adapted to house one or more of the radiation sources (3), detector (7) and/or collimator means.

21. A method of determining the concentration of glucose in blood, which comprises directing a first radiation beam of a wavelength substantially in the bandwidth 1500 to 1700nm along a first path from a source (3) to radiation detection means (7), directing a second radiation beam of wavelength substantially in the bandwidth 1200 to 1400nm, along a second path from a source (3) to said radiation detection means (7), said first and second paths passing through a test medium in a region intermediate said source(s) (3) and said detection means (7)
characterised in that the method further comprises elevating the temperature of the test medium.

22. A method according to claim 21, wherein the respective paths of the radiation beams are rectilinear as they pass through the test medium.

23. A method according to claim 22, wherein the respective paths of the radiation beams are effectively co-linear as they pass through the test medium.

24. A method according to any one of claims 21 to 23, wherein at least one of the first and second radiation beams is pulsed.

25. A method according to claim 24, wherein both the first and second radiation beams are pulsed.

26. A method according to any one of claims 21 to 25, wherein the temperature of the test medium is elevated to about 40° Celsius.

## Patentansprüche

1. Vorrichtung für die nicht-invasive Bestimmung der Glukosekonzentration im Blut eines Patienten, umfassend:
eine erste Strahlungsquelle (3) zum Erzeugen eines ersten Strahlungsbündels einer elektromagnetischen Strahlung von einer Wellenlänge mit einer Bandbreite von im wesentlichen 1500 bis 1700 nm;
eine zweite Strahlungsquelle (3) zum Erzeugen eines zweiten Strahlungsbündels einer elektromagnetischen Strahlung mit einer zweiten Wellenlänge in der Bandbreite von 1200 bis 1400 nm;
einen Detektor (7) zum Feststellen einer elektromagnetischen Strahlung aus den ersten und zweiten Strahlungsbündeln;
eine Vorrichtung (5, 6) zum Ausrichten der ersten und zweiten elektromagnetischen Strahlungsbündel entlang entsprechender Bahnen durch ein Testmedium des Blutes auf den Detektor (7);
dadurch gekennzeichnet, daß eine Heizvorrichtung zum Anheben der Temperatur des Testmediums vorgesehen ist.

2. Vorrichtung (1) nach Anspruch 1, wobei die entsprechenden Bahnen der Strahlungsbündel durch das Testmedium im wesentlichen geradlinig sind.

3. Vorrichtung (1) nach Anspruch 2, wobei die Bahnen der Strahlungsbündel effektiv co-linear durch das Testmedium sind.

4. Vorrichtung (1) nach einem vorhergehenden Anspruch, wobei Pulsiermittel für die Vorrichtung (1) derart vorgesehen sind, daß mindestens eines der Strahlungsbündel gepulst wird.

5. Vorrichtung (1) nach Anspruch 4, wobei beide Strahlungsquellen (3) gepulst werden.

6. Vorrichtung (1) nach den Ansprüchen 4 und 5, wobei das Pulsiermittel angeordnet ist, um die ersten und zweiten Strahlungsbündel abwechselnd zu pulsen.

7. Vorrichtung (1) nach einem vorhergehenden Anspruch, wobei die erste Strahlungsquelle (3) angeordnet ist, um eine Wellenlänge in der Bandbreite von 1547 nm bis 1577 nm zu erzeugen.

8. Vorrichtung (1) nach einem vorhergehenden Anspruch, wobei die zweite Strahlungsquelle (3) angeordnet ist, um eine Wellenlänge in der Bandbreite von 1295 nm bis 1305 nm zu erzeugen.

9. Vorrichtung (1) nach irgendeinem der vorhergehenden Ansprüche, wobei die ersten und zweiten Strahlungsquellen (3) Lasergeräte sind.

10. Vorrichtung (1) nach Anspruch 9, wobei die ersten und zweiten Lasergeräte Laserdioden sind.

11. Vorrichtung (1) nach den Ansprüchen 9 oder 10, wobei ein einziges Lasergerät vorgesehen ist, um sowohl das erste als auch das zweite Strahlungsbündel zu emittieren.

12. Vorrichtung (1) nach einem vorhergehenden Anspruch, wobei der Detektor (7) in Form eines einheitlichen Detektors vorliegt, der zum Feststellen der Strahlung von sowohl dem ersten als auch dem zweiten Strahlungsbündel der elektromagnetischen Strahlung dient.

13. Vorrichtung (1) nach einem vorhergehenden Anspruch, wobei der Detektor (7) angeordnet ist, um ein elektrisches Ausgangssignal in Abhängigkeit von der Intensität der auf den Detektor (7) auftreffenden elektromagnetischen Strahlung zu erzeugen.

14. Vorrichtung (1) nach Anspruch (13), wobei der Detektor (7) eine Photodiode ist.

15. Vorrichtung (1) nach einem vorhergehenden Anspruch, wobei ein Kollimator für das Ausrichten der ersten und zweiten Strahlungsbündel entlang ihrer entsprechenden Bahnen durch das Testmedium vorgesehen ist.

16. Vorrichtung (1) nach Anspruch 15, wobei der Kollimator eine optische Linse (6) und/oder ein Prisma (5) zum Ausrichten der ersten und zweiten elektromagnetischen Strahlungsbündel entlang ihrer entsprechenden Bahnen umfaßt.

17. Vorrichtung (1) nach Anspruch 15, wobei der Kollimator einen optischen Wellenleiter umfaßt.

18. Vorrichtung (1) nach einem vorhergehenden Anspruch, die ferner einen mit Wänden versehenen Behälter (2) umfaßt, in welchem das Testmedium enthalten ist.

19. Vorrichtung (1) nach irgendeinem der Ansprüche 1 bis 17, welche ferner eine Hülse (13) umfaßt, deren Form und Abmessung dazu dient, einen Finger (14) eines Patienten aufzunehmen.

20. Vorrichtung (1) nach Anspruch 19, wobei die Hülse (13) zur Aufnahme einer oder mehrerer der Strahlungsquellen (3), des Detektors (7) und/oder des Kollimators dient.

21. Verfahren zum Bestimmen der Glukosekonzentration im Blut, welches umfaßt das Ausrichten eines ersten Strahlungsbündels einer Wellenlänge mit einer Bandbreite von im wesentlichen 1500 bis 1700 nm entlang einer ersten Bahn aus einer Quelle (3) zu einem Strahlungsdetektor (7), das Ausrichten eines zweiten Strahlungsbündels einer Wellenlänge in der Bandbreite von im wesentlichen 1200 bis 1400 nm entlang einer zweiten Bahn aus einer Quelle (3) zu dem Strahlungsdetektor (7), wobei die ersten und zweiten Bahnen durch ein Testmedium in einem Bereich hindurchgehen, der zwischen der (den) Quelle(n) (3) und dem Detektor (7) liegt,
dadurch gekennzeichnet, daß das Verfahren ferner das Anheben der Temperatur des Testmediums umfaßt.

22. Verfahren nach Anspruch 21, wobei die entsprechenden Bahnen der Strahlungsbündel geradlinig sind, wenn sie durch das Testmedium hindurchgehen.

23. Verfahren nach Anspruch 22, wobei die entsprechenden Bahnen der Strahlungsbündel effektiv co-linear sind, wenn sie durch das Testmedium hindurchgehen.

24. Verfahren nach irgendeinem der Ansprüche 21 bis 23, wobei mindestens eines der ersten und zweiten Strahlungsbündel gepulst wird.

25. Verfahren nach Anspruch 24, wobei sowohl das erste als auch das zweite Strahlungsbündel gepulst wird.

26. Verfahren nach irgendeinem der Ansprüche 21 bis 25, wobei die Temperatur des Testmediums bis auf etwa 40° Celsius erhöht wird.

## Revendications

1. Appareil (1) adapté pour la mesure non invasive de la concentration en glucose dans un sang de patient comprenant:
- une première source de rayonnement (3) pour engendrer un premier faisceau de rayonnement électromagnétique d'une longueur d'onde essentiellement comprise dans la largeur de bande de 1 500 à 1 700 nm;
- une deuxième source de rayonnement (3) pour engendrer un deuxième faisceau de rayonnement électromagnétique d'une deuxième longueur d'onde comprise dans la largeur de bande de 1 200 à 1 400 nm;
- un moyen de détection (7) configuré pour détecter le rayonnement électromagnétique provenant desdits premier et deuxième faisceaux de rayonnement;
- un moyen (5, 6) configuré pour diriger lesdits premier et deuxième faisceaux électromagnétiques le long des chemins respectifs à travers un milieu d'essai dudit sang vers ledit moyen de détection (7);
caractérisé en ce qu'un moyen de chauffage (10) est prévu pour permettre d'élever la température du milieu d'essai.

2. Appareil (1) selon la revendication 1, dans lequel les chemins respectifs des faisceaux de rayonnement sont essentiellement rectilinéaires à travers ledit milieu d'essai.

3. Appareil (1) selon la revendication 2, dans lequel les chemins des faisceaux de rayonnement sont effectivement co-linéaires à travers ledit milieu d'essai.

4. Appareil (1) selon l'une quelconque des revendications précédentes, dans lequel un moyen de modulation par impulsion est fourni pour l'appareil (1), de sorte qu'au moins un des faisceaux de rayonnement est modulé par impulsion.

5. Appareil (1) selon la revendication 4, dans lequel les deux sources de rayonnement (3) sont modulées par impulsion.

6. Appareil (1) selon les revendications 4 et 5, dans lequel le moyen de modulation par impulsion est configuré pour moduler par impulsion et en alternance les premier et deuxième faisceaux de rayonnement.

7. Appareil (1) selon l'une quelconque des revendications précédentes, dans lequel la première source de rayonnement (3) est configurée pour engendrer une longueur d'onde dans la largeur de bande de 1 547 nm à 1 577 nm.

8. Appareil (1) selon l'une quelconque des revendications précédentes, dans lequel la deuxième source de rayonnement (3) est configurée pour engendrer une longueur d'onde dans la largeur de bande de 1 295 nm à 1 305 nm.

9. Appareil (1) selon l'une quelconque des revendications précédentes, dans lequel les première et deuxième sources de rayonnement (3) sont des dispositifs laser.

10. Appareil (1) selon la revendication 9, dans lequel les premier et deuxième dispositifs laser sont des diodes laser.

11. Appareil (1) selon la revendication 9 ou 10, dans lequel un seul desdits dispositifs laser est prévu pour émettre à la fois les premier et deuxième faisceaux de rayonnement.

12. Appareil (1) selon l'une quelconque des revendications précédentes, dans lequel le moyen de détection (7) est sous forme d'un détecteur unitaire disposé pour détecter le rayonnement à la fois des premier et deuxième faisceaux du rayonnement électromagnétique.

13. Appareil (1) selon l'une quelconque des revendications précédentes, dans lequel le moyen de détection (7) est configuré pour produire un signal de sortie électrique fonction de l'intensité du rayonnement électromagnétique frappant le détecteur (7).

14. Appareil (1) selon la revendication 13, dans lequel le détecteur (7) est une photodiode.

15. Appareil (1) selon l'une quelconque des revendications précédentes, dans lequel le moyen de collimation est prévu configuré pour diriger les premier et deuxième faisceaux de rayonnement le long de leurs chemins respectifs à travers le milieu d'essai.

16. Appareil (1) selon la revendication 15, dans lequel le moyen de collimation comprend une lentille optique (6) et/ou un prisme (5) configuré pour diriger les premier et deuxième faisceaux de rayonnement électromagnétique le long de leurs chemins respectifs.

17. Appareil (1) selon la revendication 15, dans lequel le moyen de collimation comprend un moyen de guide d'onde optique.

18. Appareil (1) selon l'une quelconque des revendications précédentes, qui comprend de plus un réceptacle à parois (2) dans lequel le milieu d'essai est contenu.

19. Appareil (1) selon l'une quelconque des revendications 1 à 17, qui comprend de plus un élément de manchon (13) conformé et dimensionné pour recevoir un doigt (14) d'un patient.

20. Appareil (1) selon la revendication 19, dans lequel l'élément de manchon (13) est adapté pour loger un ou plusieurs des sources de rayonnement (3), du détecteur (7) et/ou du moyen de collimation.

21. Procédé pour déterminer la concentration en glucose dans le sang, qui consiste à diriger un premier faisceau de rayonnement d'une longueur d'onde essentiellement dans une largeur de bande de 1 500 à 1 700 nm le long d'un premier chemin à partir d'une source (3) vers un moyen de détection de rayonnement (7); à diriger un deuxième faisceau de rayonnement d'une longueur d'onde essentiellement dans la largeur de bande de 1 200 à 1 400 nm, le long d'un deuxième chemin provenant d'une source (3) vers ledit moyen de détection de rayonnement (7), lesdits premier et deuxième chemins traversant un milieu d'essai dans une région intermédiaire entre ladite ou lesdites sources (3) et ledit moyen de détection (7),
caractérisé en ce que le procédé consiste de plus à élever la température du milieu d'essai.

22. Procédé selon la revendication 21, dans lequel les chemins respectifs des faisceaux de rayonnement sont rectilinéaires comme ils traversent le milieu d'essai.

23. Procédé selon la revendication 22, dans lequel les chemins respectifs des faisceaux de rayonnement sont effectivement co-linéaires comme ils traversent le milieu d'essai.

24. Procédé selon l'une quelconque des revendications 21 à 23, dans lequel au moins un des premier et deuxième faisceaux de rayonnement est modulé par impulsion.

25. Procédé selon la revendication 24, dans lequel le premier et le deuxième faisceaux de rayonnement sont tous deux modulés par impulsion.

26. Procédé selon l'une quelconque des revendications 21 à 25, dans lequel la température du milieu d'essai est élevée à environ 40°C.
